# EUROPEAN PATENT SPECIFICATION

(11) **EP 0 589 205 B1**
(45) Date of publication and mention of the grant of the patent: **25.06.2003**
(21) Application number: 93113193.2
(22) Date of filing: 14.04.1988
(51) Int. Cl.: C12N 15/52, C12N 1/21, C12P 19/06

(54) **Preparation of xanthan gum using Xanthomonas campestris having lactose utilization genes integrated into the chromosome**
Herstellung von Xanthan-Gummi mittels Xanthomonas campestris mit Genen zur hydrolisierung von Laktose integriert im Chromosom.
Préparation de gomme de xanthane en utilisant Xanthomonas campestris ayant des gènes d'utilisation de lactose intégrés dans le chromosome

(30) Priority: 14.04.1987 US 38302
(43) Date of publication of application: 30.03.1994
(62) Divisional of application: 88303352.4
(73) Proprietor: Shin-Etsu Chemical Co., Ltd., Chiyoda-ku Tokyo 100 (JP); SHIN-ETSU BIO, Inc., San Diego, California 92121 (US)
(72) Inventor: Pollock, Thomas J., San Diego, California 92122 (US); Thorne, Linda, Palomar, , California 92060 (US)
(74) Representative: Harrison, David Christopher

(56) References cited:
- EP-A- 0 233 019
- WO-A-87/05938
- ABSTRACTS OF THE ANNUAL MEETING OF THE AMERICAN SOCIETY FOR MICROBIOLOGY vol. 86 , 26 March 1986 , WASHINGTON, DC, USA page 272 N.E. HARDING ET AL. 'Genetic and physical analysis of cloned xanthan gum biosynthetic genes from Xanthomonas campestris'
- JOURNAL OF BACTERIOLOGY vol. 169, no. 8 , August 1987 , WASHINGTON, D.C. , USA pages 3593 - 3600 L. THORNE ET AL. 'Clustering of mutations blocking synthesis of xanthan gum by Xanthomonas campestris'
- JOURNAL OF BACTERIOLOGY vol. 169, no. 6 , June 1987 , WASHINGTON, D.C., USA pages 2854 - 2861 N.E. HARDING ET AL. 'Genetic and Physical Analysis of a cluster of genes essential for Xanthan Gum Biosynthesis in Xanthomonas Campestris'
- INTERNATIONAL JOURNAL OF BIOLOGICAL MACROMOLECULES vol. 8 , December 1986 , LONDON, GB pages 372 - 374 G.C. BARRERE ET AL. 'Molecular cloning of genes involved in the production of extracellular polysaccharide xanthan by Xanthomonas campestris pv. campestris'
- ABSTRACTS OF THE 193RD ACS NATIONAL MEETING vol. 193 , 10 April 1987 , WASHINGTON, D.C., USA M.R. BETLACH ET AL. 'Genetically engineered polymers: manipulation of xanthan biosynthesis'
- APPLIED AND ENVIRONMENTAL MICROBIOLOGY vol. 47, no. 2 , February 1984 , WASHINGTON, D.C., USA pages 253 - 257 P.M. WALSH ET AL. 'Genetic construction of lactose-utilising Xanthomonas campestris'
- OBUKOWICZ ET AL.: 'Integration of the delta-endotoxin gene of Bacillus thuringiensis into the chromosome of root-colonizing strains of pseudomonads using Tn5.' GENE vol. 45, no. 3, 1986, pages 327 - 331

## Description

The present invention relates to the synthesis of xanthan gum by Xanthomonas campestris and particularly to methods for increasing synthesis by modifying the natural organism.

A number of microorganisms produce extracellular polysaccharides, also known as exopolysaccharides or EPS. The exopolysaccharide known as xanthan is produced by the bacterium Xanthomonas campestris. The strain X. campestris pv campestris is a causal agent of black rot of crucifers.

Xanthan itself is useful as a specialty polymer for a growing number of commercial applications. The exploitation of xanthan as a commercial product results from a successful screening effort by the Northern Regional Research Center to find useful water-soluble polysaccharide products to replace existing gums from plant and algal sources. The NRRL discovered X. campestris NRRL B1459, a strain which produces a polymer that exhibits three desirable properties: (1) high viscosity at low concentrations; (2) pseudoplasticity; and (3) insensitivity to a wide range of temperature, pH, and electrolyte concentrations. Because of its special rheological properties, xanthan is used in food, cosmetics, pharmaceuticals, paper, paint, textiles, and adhesives and otherwise in the oil and gas industry.

In addition, the polymer is readily produced by fermentation from D-glucose. The synthesis of xanthan is believed to be similar to exopolysaccharide synthesis by other Gram-negative bacteria, such as species of Rhizobium, Pseudomonas, Klebsiella, and Escherichia. The synthetic pathway can be divided into three parts: (1) the uptake of simple sugars and their conversion to nucleotidal derivatives; (2) the assembly of pentasaccharide subunits attached to an isopentenyl pyrophosphate carrier; and (3) the polymerization of pentasaccharide repeat units and their secretion. By comparison to the more advanced molecular genetic understanding of colanic acid synthesis by E. coli or alginate synthesis by P. aeruginosa, little is known about the genes, enzymes, or mechanisms that control the synthesis of xanthan by X. campestris.

Xanthan gum is usually produced by fermentation of X. campestris with glucose or corn syrup as the major carbon source. Although it is also possible to convert the glucose and galactose in hydrolyzed cheese whey to xanthan gum, wild-type strains of X. campestris utilize lactose poorly, and the whey must first be hydrolyzed enzymatically with lactase or β-galactosidase. There are some suggestions that the β-galactosidase of X. campestris has a low affinity for lactose, thereby accounting for the poor utilization of unhydrolyzed lactose. Attempts have been made to generate a strain of X. campestris that can utilize lactose more efficiently. Exogenous lac genes have been transferred into X. campestris using transposon Tn951 which was in turn inserted within the mobilizable broad host range plasmid RP1. However, the plasmid, and therefore the lac genes, were not stable in the absence of a plasmid-selective antibiotic : (Walsh et al. Appl. Environ. Microbiol., 1984, 47(2):253-25. Other investigators isolated a spontaneous derivative of X. campestris B1459 that could convert unhydrolyzed lactose in whey to xanthan gum. However, the nature of the mutation was not known, and the strain proved to be unstable for xanthan production, losing considerable productivity within forty generations under non-selective conditions.

Other genetic manipulations of X. campestris are also desirable. For example, undesirable enzymes are sometimes produced that contaminate the xanthan product, limiting the usefulness of xanthan gum to a narrower range of situations than would otherwise be possible.

Accordingly, an increased understanding of the genetic control of xanthan production by X. campestris would be useful for improving the productivity of X. campestris for xanthan synthesis.

A recent publication on the topic of molecular cloning of genes involved in the production of xanthan in Barrere et al., Int. J. Biol. Macromol. (1986) 8: 372-374. A study showing that a mutation, which blocks exopolysaccharide synthesis and prevents nodulation of peas by Rhizobium leguminosarum, was corrected by cloned DNA from the phytopathogen Xanthomonas is described in Borthakur et al., Mol. Gen. Genet. (1986) 203:320-323. Production of xanthan using Xanthomonas campestris, properties of xanthan, and commercial applications of xanthan are described in Rogovin et al., J. Biochem. Microbiol. Technol. Eng. (1961) 3:51-63, and Kennedy et al., 1984, "Production, properties, and applications of xanthan", pp. 319-371 in M.E. Bushell (ed.), Progress in Industrial Microbiology, vol. 19, Elsevier, Amsterdam.

A number of publications have occurred after the filing of U.S. Application Serial No. 038,302 on April 14, 1987. These include Harding et al., J. Bacteriol. (1987) 169:2854-2861, which describes genetic and physical analyses of a cluster of genes essential for xanthan gum biosynthesis in X. campestris. European Patent Application EP 0 233 019 A2, filed January 29, 1987, describes a recombinant DNA plasmid for xanthan gum synthesis. Thorne et al., J. Bacteriol. (1987) 169:3593-3600, describes clustering of mutations blocking synthesis of xanthan gum by X. campestris.

The invention provides a method of increasing xanthan gum production which comprises culturing a Xanthomonas campestris strain having a xanthan-increasing modification in a culture medium, wherein said modification is expressible exogenous genetic information controlling the synthesis of xanthan; and separating xanthan from the culture medium. A section of Xanthomonas chromosomal DNA containing genetic Information controlling the synthesis of xanthan is identified, which allows use of numerous techniques for increasing xanthan production such as providing multiple copies to increase xanthan production by a dosage effect and providing an inducible promoter or other method of genetic control in order to decouple xanthan production from constitutive protein synthesis.

### BRIEF DESCRIPTION OF THE DRAWINGS

The present invention will be better understood by reference to the following detailed description of specific embodiments when considered in combination with the enclosed drawings which form part of the specification, wherein:

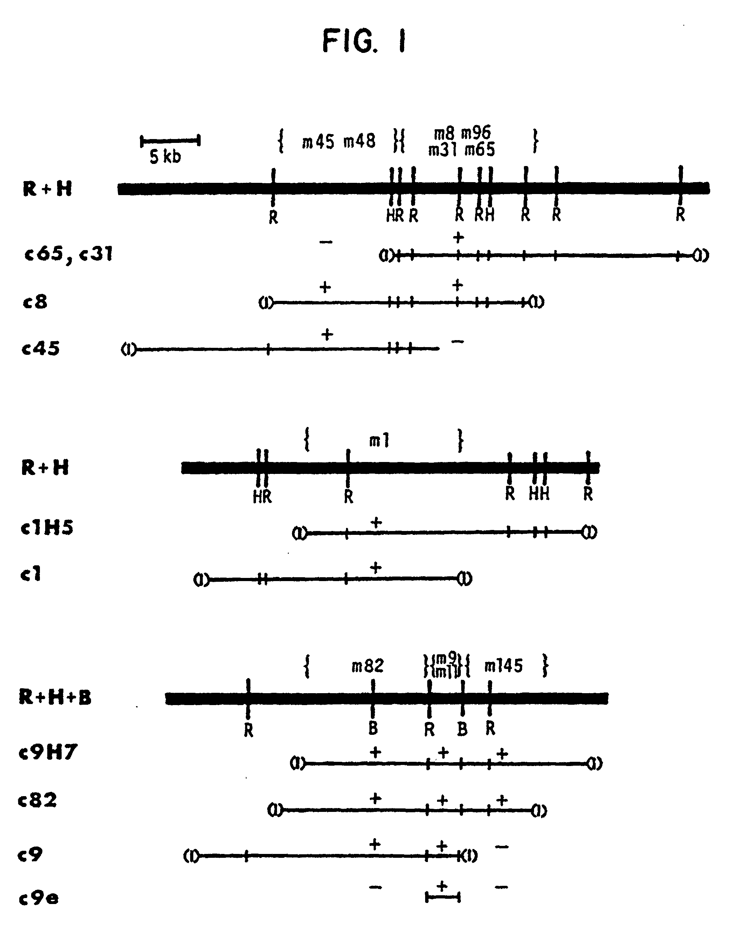

Figure 1 is a compilation of three physical maps for X. campestris DNA insertions in vector pRK311 showing complementation groups. The line marked "R/H" shows the order and position of restriction cleavage sites for EcoRI and HindIII enzymes deduced from the overlapping maps obtained for individual cloned inserts. Parentheses, (), at the end of the cloned maps indicate that it was not possible to distinguish between an end generated by cleavage within the cloned insert from restriction in the adjoining multiple cloning site. The tentative map positions for Xgs⁻ mutations are indicated above the physical maps. Unordered loci are enclosed with braces, {}.

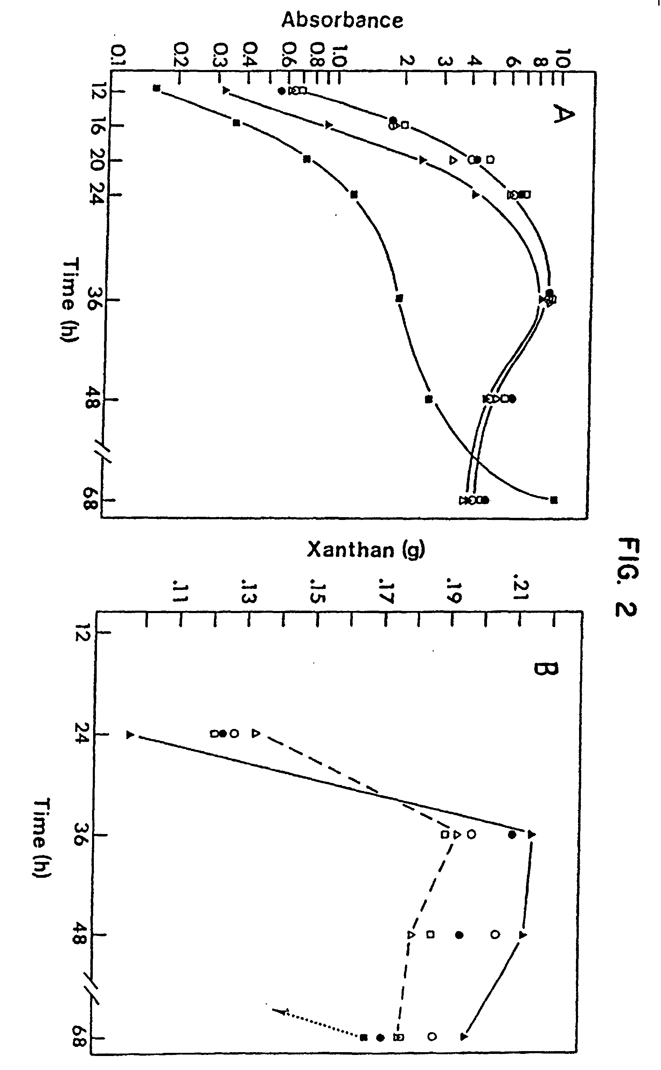

Figure 2 is-a graph showing time course of accumulation of xanthan by wild-type strain X59 carrying multiple copies of genetic information controlling the synthesis of xanthan. Recombinant plasmids containing inserts of cloned X. campestris DNA that restore xanthan synthesis are indicated by the following symbols: ● , X59; Δ , X59pRK311; ▲ , X59c45; □ , X59c9; ○ , X59c1; ■ , X59c31. Panel A shows optical density at various times of cell growth while Panel B shows xanthan accumulation. The upper curve in Panel A represents four cultures. In Panel B the solid line is for X59c45, the dashed line is for X59pRK311, and the dotted line is for X59c31.

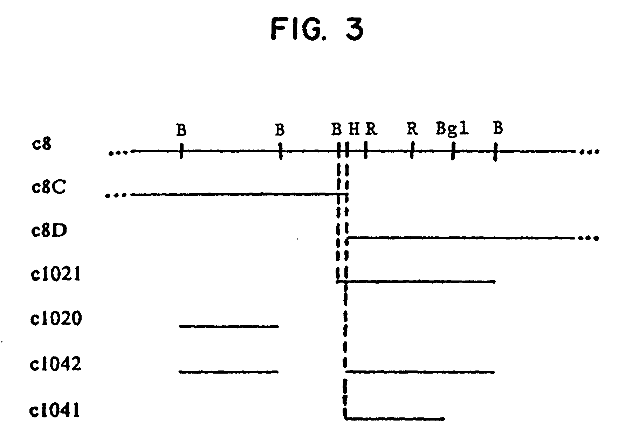

Figure 3 is a restriction map showing subcloned fragments of the c8 fragment of X. campestris DNA shown in Figure 1. Abbreviations: B, BamHI; Bgl, BglII; H, HindIII; R, EcoRI.

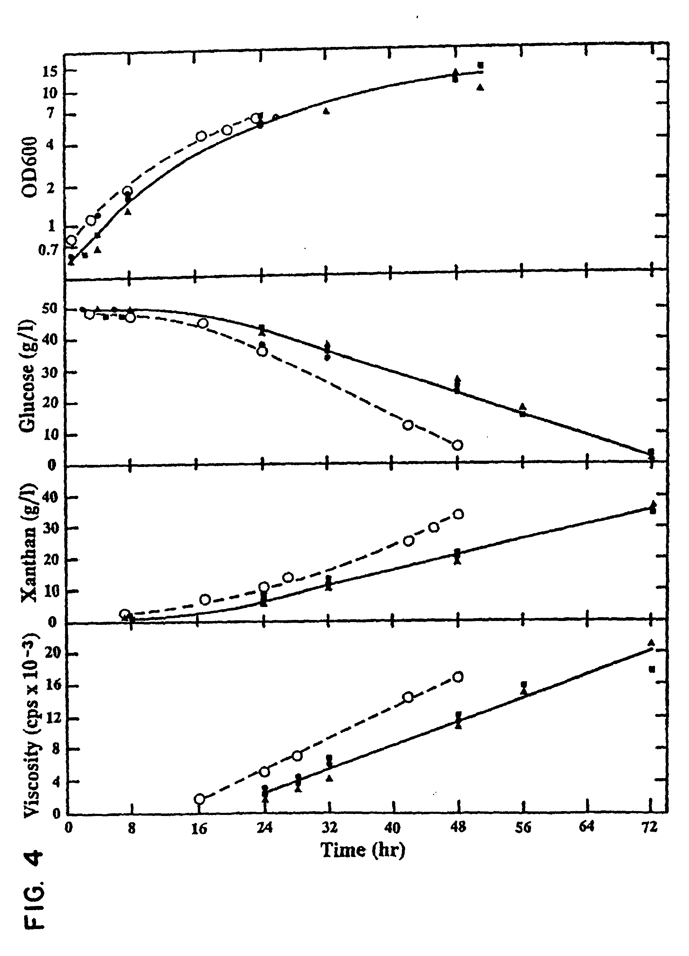

Figure 4 is a graph with four panels showing four different characteristics of three control cultures in comparison to a rifampicin-resistant strain, X59.

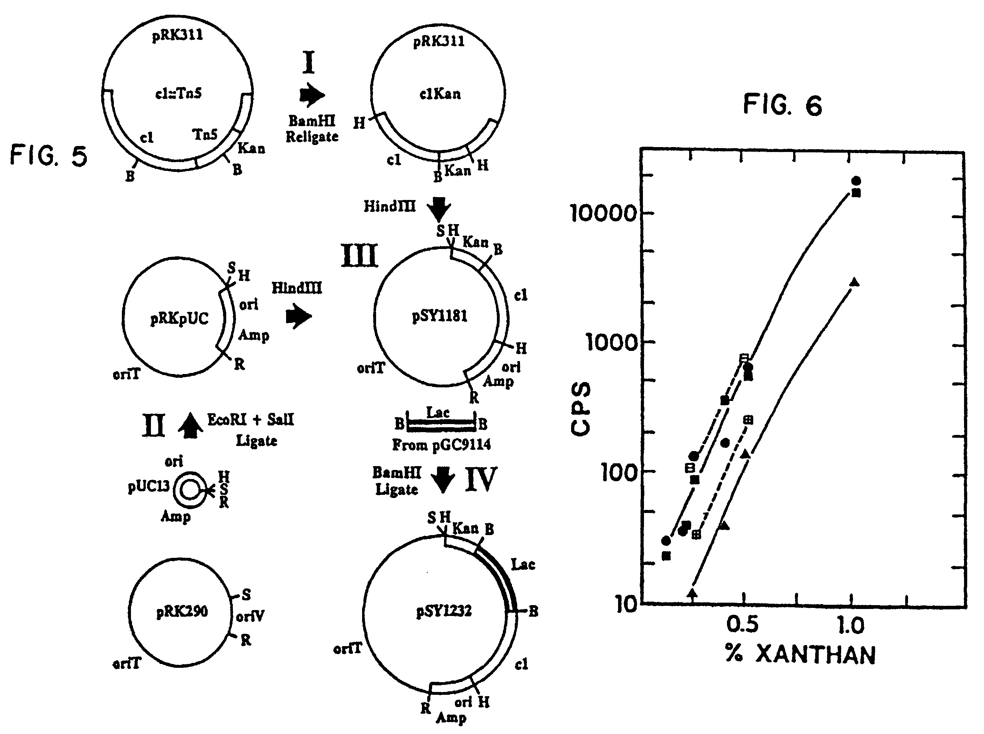

Figure 5 is a schematic diagram showing construction of a Lac⁺ integration vector. The circular genetic maps are drawn roughly to scale. Plasmid pSY1181 is a general purpose integration vector and carries a DNA segment (c1) that is identical to an X. campestris chromosomal sequence. Plasmid pSY1232 carries in addition the lac genes from Tn951.
Figure 6 is a graph showing viscosity of xanthan-containing material made from glucose, lactose, or clarified whey. Solutions of xanthan gum were prepared at defined concentrations and to the exclusion of water, ash, and protein. Viscosities at different shear rates were measured, and the values from a shear rate of 1.32 sec⁻¹ were plotted. Symbols, strains and growth or processing conditions: ● , X59 (Lac⁻), glucose; ■ , X59-1232 (Lac⁺), lactose; ▲ , X59-1232 (Lac⁺), clarified whey; 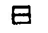 , X59-1232 (Lac⁺), lactose, without clarified whey added at harvest; 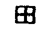 , X59-1232 (Lac⁺), lactose, with clarified whey added at time of harvest.

### DESCRIPTION OF SPECIFIC EMBODIMENTS

Investigations in the laboratories of the inventors have indicated that a number of modifications are available that are capable of increasing xanthan gum production from Xanthomonas campestris strains. Three specific genetic modifications capable of increasing xanthan production are mutations causing rifampicin-resistance, mutations causing bacitracin-resistance, and the presence of exogenous genetic information controlling the synthesis of xanthan introduced into a Xanthomonas campestris strain.

The first two of these techniques, both of which involve utilization of a mutant strain having resistance to an antibiotic, can be carried out in a straightforward manner now that the relationship between antibiotic resistance and xanthan production has been determined; they are disclosed and claimed in EP-A-287363 from which the present application is divided. The present application is concerned with the third technique.

This third technique for increasing xanthan production involves the use of exogenous genetic information controlling the synthesis of xanthan that has now been identified. Specific sections of Xanthomonas chromosomal DNA have been identified that control the synthesis of xanthan. Figure 1 is a chromosome map providing restriction site information that is useful in identifying the proper sequences. Three deposits of genetic information have also been made (April 10, 1987) with the American Type Culture Collection, Rockville, Maryland, U.S.A., under the Budapest Treaty on the International Recognition of the Deposit of Microorganisms for the Purposes of Patent Procedure. The three deposits are E. Coli strains containing (individually) the three genetic segments identified in Figure 1. These deposits have been accorded deposit numbers ATCC 67386, ATCC 67387, and ATCC 67388.

The genetic information from X. campestris can be utilized in many ways. Plasmids can be constructed containing the exogenous genetic information controlling the synthesis of xanthan, and genetic information can be introduced into an X. campestris host by utilizing a donor strain containing a plasmid with the desired genetic information in a triparental mating scheme to transfer the genetic information to X. campestris. Suitable vectors for conjugation include a variety of plasmids displaying a broad host range. For example, IncP-group plasmids, which include RK2 and its derivatives pRK290, pLAFR1, and pRK311, and IncQ-group plasmids, which include RSF1010 and its derivative pMMb24 can be utilized. References describing these plasmids include Ditta et al., Plasmid (1985) 13:149-153 (plasmids RK2, pRK290, and pRK311); Friedman et al., Gene (1982) 18:289-296 (pLAFRI); and Bagdasarian et al., Gene (1983) 26:273-282 (pMMb24). For example, E. coli donor cells containing the genetic information on a plasmid, E. coli HB101 helper cells containing plasmid pRK2013, and recipient X. campestris cells can be incubated to cause genetic information transfer by conjugation. Isolation of recombinant plasmids, for example by utilization of a marker present adjacent to the genetic information being transferred, can be followed by further purification and subsequent matings. With a purified member of the original gene library to raise the frequency of exconjugates containing the exogenous genetic information.

Individual genes encoding specific peptides or control factors utilized in the synthesis of xanthan can be isolated from the genetic information described above using standard techniques of recombinant DNA technology. Restriction endonucleases can be utilized to cleave the relatively large segment of genetic information containing xanthan genes into specific identifiable fragments. These fragments can be individually cloned and identified. Individual fragments can be inserted into new hosts to provide further X. campestris strains having increased xanthan production. Accordingly, fragments of the genetic information controlling the synthesis of xanthan also have utility in the commercial production of xanthan. The phrase "genetic information controlling the synthesis of xanthan" accordingly refers either to the original chromosomal DNA that controls xanthan synthesis as described above or fragments of this original chromosomal DNA containing one or more individual genes capable of controlling the synthesis of xanthan (e.g., individual genes encoding an enzyme utilized in xanthan synthesis).

Specific examples showing manipulation of the genetic material are set forth in the working examples which follow. Clones containing fragments of the genetic information controlling the synthesis of xanthan have been prepared. Selection among these clones has allowed isolation of strains with improved properties, such as increased viscosity. Genetic material has been transferred in combination with known genetic material to produce modified strains capable of being utilized in manners not previously available. For example, genetic material of the invention has been transferred along with genetic information controlling production of lactase to provide a strain that is particularly suitable for use in preparing xanthan gum using cheese whey as a substrate.

For example, a stable recombinant strain, X59-1232, which produces quality xanthan gum from lactose, was obtained by inserting the lac genes from transposon Tn951 into the chromosome of X. campestris strain X59. The apparent conversion efficiency of lactose to xanthan gum by this strain was equivalent to the conversion of glucose to xanthan gum by strain X59, a strain discussed further herein which efficiently carries out the latter conversion. The viscosities of the xanthan gums from these two sources were equivalent. After more than fourteen generations of growth without positive selection for genetically linked traits, the apparent conversion efficiency from lactose by strain X59-1232 was superior to that of strain X59-pGC9114, a strain which carries the Tn951 lac genes on a multicopy plasmid (pGC9114) which, in turn, was superior to strain X59, which lacks the Tn951 lac genes. The superiority for X59-1232 could be attributed to the stable integration of the lac genes into the chromosome of X59-1232.

The apparent efficiency of conversion for clarified cheese whey to xanthan gum by X59-1232 was approximately 90% that of lactose to xanthan gum. This 10% difference from theoretical is probably within the experimental error of the measurements employed.

After a Xanthomonas strain having a xanthan-increasing modification is cultured, xanthan is separated from the culture medium utilizing any technique capable of achieving this result such as the standard techniques already being utilized commercially. See, for example, Kennedy et al., supra. and Rogovin et al., supra. One simple technique involves filtering a liquid culture medium to remove growing bacterial cells, adding isopropyl alcohol to the filtrate to precipitate the exopolysaccharides, and collecting the precipitate on a filter followed by drying (optionally with heat and/or under a vaccuum).

The invention now being generally described, the same will be better understood by reference to the following detailed examples which are provided for purposes of illustration only and are not to be considered limiting of the invention unless so stated.

### EXPERIMENTAL

### Example 1

### Use of Exogenous Genetic Information Controlling the Synthesis of Xanthan

In summary, mutations that block the synthesis of xanthan gum by Xanthomonas campestris B1459S-4L-II were isolated as nonmucoid colonies after treatment with ethylmethane sulfonate and used to identify DNA fragments containing xanthan genes. Complete libraries of DNA fragments from wild-type X. campestris were cloned into E. coli using a broad host range cosmid vector and then transferred into each mutant strain by conjugal mating. Cloned fragments that restored xanthan gum synthesis (Xgs⁺; mucoidy) were characterized according to restriction pattern, DNA sequence homology and complementation of a subset of Xgs⁻. Groups of clones that contained overlapping homologous DNA were found to complement specific Xgs⁻ mutations. The results suggested a possible clustering of genetic loci involved in synthesis of xanthan. Other apparently unlinked loci were also discovered. Two forms of complementation were observed. In most instances, independently isolated cosmid clones that complemented a single mutation were found to be partially homologous. Less frequent was the second form of complementation, where two cosmid clones that lack any homologous sequences restored the mucoid phenotype to a single mutant. Restoration of the wild-type mucoid phenotype was shown, in the one case that was studied in detail, to coincide with homologous recombination between a normal cloned DNA residing on a plasmid and the mutant chromosomal locus. Lastly, the degree of restoration of xanthan synthesis was measured for the complemented mutants and for wild-type X. campestris carrying multiple copies of the cosmid clones. Details of experiment techniques and results are set forth below.

### Materials and Methods

### Bacterial Strains and Plasmids

Xanthomonas campestris B1459S-4L-II (our strain X55) obtained from the Northern Regional Research Center was the Xgs⁺ (xanthan gum synthesis positive) parent of all our X. campestris strains. Strain X59 was a spontaneous rifampicin-resistant derivative of X55 that was also fully Xgs⁺. Rif^{r} derivatives of X55 arose at a frequencey of about 10⁻⁹ and were selected on agar plates containing Luria broth supplemented with rifampicin at 60 µg/ml. Bacteriophage λ b221 rex::Tn5 cI857 Oam29 Pam80 (Ruvkun et al., Nature (1981) 289:85-88) was the source of Tn5 for mapping by insertional gene inactivation. Strain LE392 was the permissive host for propagating the phage. All strains and plasmids are listed in Table 1.

**Table 1**

| Bacterial Strains and Plasmids | | |
|---|---|---|
| Name | Genotype or Phenotype^{a} | Reference or Source |
| X. campestris | | |
| X55 | Xgs⁺, prototroph | B1459S-4L-II |
| X59 | Xgs⁺, prototroph, Rif^{r} | This Example |
| X59m1 | Xgs⁻, prototroph, Rif^{r} | This Example |
| X59m8 | Xgs⁻, auxotroph, Rif^{r} | This Example |
| X59m9 | Xgs⁻, prototroph, Rif^{r} | This Example |
| X59m11 | Xgs⁻, auxotroph, Rif^{r} | This Example |
| X59m31 | Xgs⁻, prototroph, Rif^{r} | This Example |
| X59m45 | Xgs⁻, prototroph, Rif^{r} | This Example |
| X59m48 | Xgs⁻, auxotroph, Rif^{r} | This Example |
| X59m65 | Xgs⁻, prototroph, Rif^{r} | This Example |
| X59m82 | Xgs⁻, prototroph, Rif^{r} | This Example |
| X59m96 | Xgs⁻, auxotroph, Rif^{r} | This Example |
| X59m145 | Xgs⁻, prototroph, Rif^{r} | This Example |

| E. coli | | |
|---|---|---|
| HB101 | F⁻ hsd20 (r_{B}⁻ m_{B}⁻), recA13, ara-14, proA2, lacY1, galK2, rpsL20 (streptomycin-resistance), xy1-5, mt1-1, supE44, thi, leu, λ⁻ | Bethesda Research Labs |
| JM109 | recA1, endA1, gyrA96, thi, hsdR17, supE44, relA1, Δ(lac-proAB), [F'traD36, proAB, lacI^{q}ZΔM15] | Bethesda Research Labs |
| LE392 | F⁻, hsdR514, (rₖ⁻mₖ⁻), supE44, supF58, λ⁻, galK2, galT22, metB1, trpR55, lacY1, Δlac IZY-6 | L. Enquist |
| Bacteriophage | λb221 rex::Tn5 (Kan^{r}) cI857, Oam29, Pam80 | Ruvkun et al. Nature (1981) 289:85-88 |

| Plasmids | | |
|---|---|---|
| pRK311 | RK2 origin, Tra⁺, Mob⁻, Tet^{r}, λcos, lacZ(∝) | Ditta et al. Plasmid (1985) 13:149-153 |
| pRK2013 | ColE1 origin, Imm⁺, Amp^{r}, Tra⁺, Mob⁺, Kan^{r} | Figurski et al. Proc Natl Acad Sci USA (1979) 76:1648-1652 |
| pUC13 | Amp^{r}, ColE1 origin | Veira et al, Gene (1982) 19:259-268 |
| c1 | pRK311, Tet^{r}, complements m1 | This Example |
| c8 | pRK311, Tet^{r}, complements m8 | This Example |
| c8::Tn5- 1- +20 | Tet^{r}, Kan^{r} | This Example |
| c9 | pRK311, Tet^{r}, complements m9 | This Example |
| c31 | pRK311, Tet^{r}, complements m31 | This Example |
| c45 | pRK311, Tet^{r} complements m45 | This Example |
| c65 | pRK311, Tet^{r} complements m65 | This Example |
| c82 | pRK311, Tet^{r} complements m82 | This Example |
| c1H5 | pRK311, Tet^{r} complements m1 | This Example |
| c9H7 | pRK311, Tet^{r} complements m9 | This Example |
| c9e | pRK311, Tet^{r} complements m9 | This Example |

| | | |
|---|---|---|
| ^{a} Abbreviations: Xgs⁺, xanthan gum synthesis; Rif^{r}, rifampicin resistance; Tet^{r}, tetracycline resistance; Kan^{r}, kanamycin resistance; Amp^{r}, ampicillin resistance; Imm⁺, colicin E1 immunity; Tra and Mob, transfer and mobilization functions of RK2 plasmid. | | |

### Growth Media

Xanthomonas species were cultured by shaking in liquid YT medium at 30°C with rifampicin at 50 µg/ml, tetracycline at 7.5 µg/ml and/or kanamycin at 50 µg/ml added for plasmid maintenance. YT medium contains Bacto tryptone (16 g/l) Bacto yeast extract (10 g/l) and NaCl (5 g/l). All nutrient agar plates contained TBAB (tryptose blood agar base from Difco) plus starch at 1% (w/v). Selection plates for conjugal matings contained tetracycline at 7.5 µg/ml, kanamycin at 50 µg/ml and rifampicin at 50 µg/ml. Minimal agar plates contained M9 inorganic salts (Anderson, Proc. Natl. Acad. Sci. USA (1946) 32:120-128) plus glucose, mannose or fructose at 1% (w/v) as the carbon source. Liquid medium for shake flask experiments to measure xanthan accumulation was referred to as "XG004" and consisted of 1X basic salts, 0.5% (w/v) tryptone, 0.25% (w/v) yeast extract, 1X trace minerals, 0.01% (w/v) CaCl and 2% (w/v) glucose. 10X basic salts consists of 6.8 g KH₂PO₄, 0.2 g MgSO₄·7H₂O, 2.2 g L-glutamic acid, 2 g citric acid in 100 ml with pH adjusted to 7 with NaOH at 30°C. 1000X trace minerals was 2.25 g FeCl₃·6H₂O, 1.41 g MnSO₄·H₂O, 2.2 g ZnSO₄·7H₂O, 0.25 g CuSO₄·5H₂O, 0.4 g CoCl₂·6H₂O, 0.26 g Na₂MoO₄·2H₂O, 0.4 g H₃BO₃ and 0.06 g KI per liter of deionized H₂O (with HCl added to solubilize the salts). E. coli was grown in Luria broth at 37°C with tetracycline at 10µg/ml and kanamycin at 50 µg/ml as appropriate or on agar plates containing Luria broth or TBAB (Difco).

### Mutagenesis of X. campestris

About 2x10⁹ freshly grown cells (an absorbance at 600 nm of 1 equals 10⁹ X. campestris cells) were resuspended in 2 ml of minimal salts medium and shaken at 30°C with 0 to 40 µl of ethylmethane sulfonate (EMS) for 1, 2 or 3 h. Samples of 0.5 ml were taken from each treatment, washed two times with YT medium and resuspended in 2 ml of YT medium and shaken overnight at 30°C. Dilutions were spread on TBAB plus 1% (w/v) starch plates. After three days, nonmucoid colonies (about 1% of the total) were saved. The mutants designated X59m1 to X59m150, were tested for retention of the Rif^{r} marker of the parent X59, for the presence of cleared zones around colonies on plates containing starch and for ability to utilize different carbon sources.

### DNA Isolation and Recombinant DNA Techniques

Plasmid DNA was isolated by the boiling method of Birnboim and Doly (Nucleic Acids Res. (1979) 7:1513-1523). Frequently used plasmids were further purified by equilibrium sedimentation in density gradients of CsCl containing ethidium bromide (Maniatis et al. 1982. Molecular Cloning, A Laboratory Manual. Cold Spring Harbor Laboratory, Cold Spring Harbor, NY). Restriction enzymes (from Boehringer Mannheim, GmbH) were used according to the manufacturer's instructions. DNA sequence homology was demonstrated by the blotting method of Southern (Maniatis et al., supra) and used Zeta-probe (Bio-Rad) for DNA immobilization. DNA for use as a hybridization probe was labeled with [³²P]dCTP (using a nick translation reagent kit from Bethesda Research Laboratories). Fragments of DNA were separated by electrophoresis through agarose gels (0.6 to 0.7% w/v) in Tris-acetate buffer (Maniatis et al., supra).

### Conjugation and Complementation of Xgs⁻ Mutants

The complete library (or specific elements of the library) were transferred from E. coli to X. campestris by a triparental mating scheme (Ditta et al., Proc. Natl. Acad. Sci. USA (1980) 77:7347-7351). From fresh overnight cultures, 10⁹ recipient cells (X. campestris Xgs⁻ mutants), 5x10⁸ donor cells (JM109-L[X59], the library) and 5x10⁸ helper cells (E. coli HB101 containing plasmid pRK2013) were mixed and passed through an HA 0.45 micron Millipore filter. The filters were incubated on TBAB plates overnight at 30°C and then the cells were washed into 2 ml of selecting medium (TBAB plus tetracycline at 7.5 µg/ml and rifampicin at 50 µg/ml). The cells were diluted by 10⁴ to 10⁵ fold and spread on selection plates containing antibiotics. Complementation (restoration of the Xgs⁺ phenotype in an Xgs⁻ mutant) occurred at a frequency of 0.1 to 0.5%. The Xgs⁺ exconjugants were purified and the recombinant plasmid was isolated and transferred back to E. coli JM109 for storage and further purification. Subsequent matings with a purified member of the library raised the Xgs⁺ frequency in the exconjugants to 100%.

### Measurement of Xanthan Accumulation

Strains to be tested were grown in liquid XG004 medium overnight, diluted, and resuspended at the same cell density. Flasks (125 ml capacity) containing 10 ml of medium XG004 were inoculated with equal numbers of cells (1x10⁸) and shaken at 28°C at 250 rpm. At the time of sampling, 20 ml of isopropyl alcohol was added to each flask to precipitate the exopolysaccharides. The precipitate was collected on a GFA filter, which was then dried in a vacuum oven, and weighed.

### Results

### Isolation of Mutants Deficient in Xanthan Gum Synthesis (Xgs⁻)

Strain X55 (NRRL B1459S-4L-II from the Northern Regional Research Center) is the "wild-type" parent of most xanthan-producing strains of X. campestris in use today. Strain X55 was the parent of all other X. campestris used in this work. A spontaneous Rif^{r} derivative of X55 was isolated by spreading about 10⁹ bacteria on a plate containing rifampicin at 60 µg/ml. The Rif^{r} phenotype of X59 was useful as a marker to distinguish progeny from contaminants following mutagenesis and as a counterselection for E. coli Rif^{s} donors in conjugal matings. Both X55 and X59 form indistinguishable mucoid colonies on nutrient and minimal agar plates.

A collection of Xgs⁻ mutants was generated by exposing strain X59 (and less frequently X55) to ethylmethane sulfonate (EMS). After growth at 30°C for 3 d, nonmucoid colonies were selected and purified for further use. In most cases the nonmucoid colonies were distinctively different in appearance, but some independently isolated mutants displayed similar nonmucoid appearance. The latter could be distinguished by plating on different carbohydrate sources and as a function of time of growth. Only one mutant was selected from each treatment with EMS, unless colony morphology was clearly distinctive. Mutants of X59, serially designated X59m1 to X59m200, were tested for the parental Rif^{r} marker. Other indications that a survivor of mutagenesis was X. campestris, an amylase producer, was the clear zone surrounding colonies spread on a nutrient agar plate containing starch and the characteristic yellowish pigment of the colony. Many of the mutants were also tested for their ability to grow on minimal agar plates containing various sugar substrates in order to distinguish unique isolates from siblings.

### Cloning of X. campestris DNA into a Cosmid Vector

Total DNA from strain X59 (Xgs⁺) was prepared by the boiling method of Birnboim and Doly, supra, and partially digested with Sau3A restriction endonuclease. Large fragments of 20 to 30 kb were purified by velocity sedimentation in neutral sucrose gradients. This ensured that only contiguous chromosomal DNA fragments were inserted in the cloning vector upon ligation. The cloning vector was the broad host range cosmid, pRK311, constructed by Ditta et al., supra. DNA fragments to be cloned were inserted into the BamHI sequence of the multiple cloning site within the lacZ portion of the vector. Using the in vitro packaging kit of Strata-gene, we selected for insertions of DNA of about 20 to 25 kb into the cosmid vectors. The pRK311 vector also carries a selectable tetracycline-resistance gene. After in vitro ligation and packaging, E. coli JM109 was transfected with phage particles, and tetracycline-resistant colonies were individually saved. Each tetracycline-resistant colony contained the plasmid vector plus a 20 to 25 kb insertion of X. campestris DNA. A library of fragments of DNA resulted from pooling the clones. Since the number of clones in each library exceeded 1000 we were at least 99.9% certain of having all fragments of the X. campestris chromosome represented at least once. Three different libraries were used in this example.

### Complementation of Xgs⁻ defects by cloned normal DNA

Intergenic conjugal matings were used to transfer DNA. The RK2-derived pRK311 cosmid has a broad host range but is not self-transmissible. In order for pRK311 1 to be transferred by conjugation between E. coli and X. campestris a second "helper" plasmid was used, pRK2013, which has a limited host range that does not include X. campestris. Transfer of recombinant cosmids was accomplished by a triparental mating that included E. coli JM109/pRK311, JM109/pRK2013 and the recipient X. campestris Xgs⁻ mutant.

About 15 different Xgs⁻ mutants were complemented and restored to mucoidy (Xgs⁺) by conjugal mating with the complete library of X. campestris genes. The frequency of complementation was about 0.1% for most matings, as would be expected if there was only one copy of each gene per chromosome. The results can be understood by considering a set of related colonies: X59-pRK311, X59m45-pRK311 and X59m45-c45. The presence of the c45 cosmid restores the mucoid appearance as seen for the wild-type X59. The mucoid phenotype for X59m45-c45 depended on the continued presence of the recombinant plasmid, as judged by the maintenance of the Tet^{r} gene of the plasmid. A similar overall pattern was seen for all complemented Xgs⁻ mutants. Several mucoid exconjugants were picked and purified by replating. DNA was prepared for each and transformed into E. coli and then mated back into the original mutant Xgs⁻ mutant strain. In each case this resulted in 100% complementation and all of the tetracycline-resistant exconjugants carried the same recombinant cosmid. The transformants of E. coli also served as a source of DNA for restriction mapping and DNA hybridization tests by Southern blotting. A summary of the complementation data is included in Table 2.

**Table 2**

| Complementation of Xgs⁻ Mutations by Wild-Type Cloned X. campestris DNA^{a} | | | | | | | | |
|---|---|---|---|---|---|---|---|---|
| Mutant | Cloned Fragment | | | | | | | |
| | c1 | c8 | c9 | c11 | c31 | c45 | c65 | c82 |
| m | + | +/- | - | - | +/- | - | +/- | - |
| m8 | - | + | - | - | + | - | + | - |
| m9 | - | +/- | + | + | - | - | - | + |
| m11 | - | +/- | + | + | - | - | - | + |
| m31 | - | + | - | - | + | - | + | - |
| m45 | - | + | - | - | - | + | - | - |
| m48 | - | + | - | - | - | + | - | - |
| m65 | - | + | - | - | + | - | + | - |
| m82 | - | +/- | + | + | +/- | - | +/- | + |
| m96 | - | + | - | - | + | - | + | - |
| m145 | - | - | - | NT | - | - | - | + |

| | | | | | | | | |
|---|---|---|---|---|---|---|---|---|
| ^{a} Xgs⁻ mutants that received a recombinant cosmid by mating were scored for mucoid (+) or nonmucoid (-) appearance by visual inspection of colonies. A +/- designation indicates a partial mucoidy. NT; not tested. | | | | | | | | |

### Alignment of Cloned Inserts and Xgs⁻ Mutations by Restriction Mapping, DNA Hybridization and Genetic Complementation

When more than one recombinant plasmid from the complete library complemented the same mutation, we usually found that they were either indistinguishable sibling clones or shared considerable DNA homology. DNA hybridization analyses demonstrated this point. Several recombinant plasmids were digested with a mixture of EcoRI and HindIII enzymes. HindIII cleaves in the multiple-cloning-site to one side of the cloned insert and EcoRI cleaves to the other side and also within the vector. This produces two fragments of vector DNA of about 8 and 13 kbp. The digestion products were separated by electrophoresis through agarose gels and two samples (A and B) containing the same digestion products were analyzed. Most of the visible bands from ethidium bromide staining were X59 DNA. The hybridization probes for the A sample were radiolabeled c45 plasmid and for the B sample, the plasmid c31. The hybridization pattern indicated that cosmids c8, c31, c45 and c65 carry overlapping segments of chromosomal DNA. The region in common between these four clones includes restriction fragments of 0.6 and 1.2 kb and part of the 4.3 kb fragment.

Additional hybridization results were obtained from a separate but similar analysis. Plasmid DNA was purified, restricted with a mix of EcoRI and HindIII enzymes and fragments were separated by agarose gel electrophoresis and then transferred to filters. In a first sample the probe was radiolabeled c9H7 and in a second sample, c1H5. The hybridization pattern for the first sample showed homology between c9H7, c9 and c145, but not between c9H7 and c45-2, c45-1, c8, c31, c32b, c1 or c1H5. The pattern of the second sample showed that c1H5 is homologous only to c1. Cosmid c1H5 was initially selected from the library of cloned fragments because it hybridized to c1. The hybridization results were the basis for compiling the map shown as Figure 1.

The deduced locations of Xgs⁻ mutations are shown in Figure 1 above the map of EcoRI and HindIII restriction sites labeled "R/H" on the left. Mutants enclosed by braces have not been ordered with respect to each other. Overlapping cloned fragments could be aligned according to restriction pattern and DNA homology. Superimposed on this alignment are the results of complementation experiments with "+" signifying restoration of the Xgs⁺ phenotype to an Xgs⁻ mutant. The range of possible map positions for each mutant was then determined from the boundaries of each cloned fragment. Most of the mutants were distributed across a contiguous stretch of about 40 kbp, representing about 2% of the chromosome of X. campestris.

Two other unlinked loci involved in xanthan synthesis were also identified. One locus is represented by four overlapping cloned fragments carried on cosmids c9, c11, c9H7 and c82. All four restore the Xgs⁺ mucoid phenotype to the independent mutants m9, m11 and m82. Another pair of cosmids (c1 and c1H5) share homology with each other, but not with either of the other two sets.

### Xanthan Synthesis by Exconjugants of X59 with Multiple Copies of Complementing Cloned Genes

We transferred each complementing clone by mating into X59, already Xgs⁺, and measured xanthan synthesis. For a control we used X59 bearing the vector alone, pRK311. The cells were grown in shake flasks at 30°C, starting from inocula of 10⁷ cells per ml. The amount of xanthan was determined by standard methods: precipitation of the exopolysaccharides by two volumes of isopropanol, drying and weighing. For most complementing clones the extra gene copies had no detectable effect or caused a decrease in xanthan yield. For those that accumulated amounts of xanthan significantly higher than the control, the xanthan and cell growth data are given in Figure 2. In no case was the increase in accumulation greater than 20%. However, the rate of xanthan accumulation between 24 and 36 hrs for X59-c45 was twice that for the control X59-pRK311. When X59 without pRK311 was included in the time course experiments we found that the large vector plasmid itself had a negative effect on xanthan synthesis (data not shown). In a similar experiment X59-c8 produced an average of 22% more xanthan gum than its parent strain X59 (48-hr growth period).

This Example demonstrates that all of the three complementary regions described in Figure 1 containing xanthan genes are useful in the preparation of strains showing increased xanthan production. Reproducible changes in xanthan accumulation were observed with the introduction of exogenous genetic information, but the magnitude of change was small, plus or minus about 15%. Suppression of xanthan production was caused by the large plasmid vector itself, which depressed cell growth and xanthan synthesis. Use of other plasmid vectors should improve strain productivity.

### Example 2

### Subcloning of c8 Fragment and Resulting Xanthan Production

The "c8" fragment of X. campestris DNA was further subcloned to localize the beneficial genetic traits. The subcloned portions are diagrammed in Figure 3, relative to c8 as given in Figure 1.

Each subclone was inserted in the vector used for most of this work, pRK311, and transformed first into E. coli and then conjugally mated from E. coli to X. campestris strains X55, X59 and X50. Cell growth and xanthan accumulation were measured in 100-500 ml shake flasks with nutrient medium containing per liter of tap water: 10 g peptone, 20 g glucose., 3.5 g K₂HPO₄, 2.6 g KH₂PO₄, 0.26 g MgSO₄·7H₂O, 6 mg H₃BO₃, 6 mg ZnO, 2.6 mg FeCl₃·6H₂O, 20 mg CaCO₃ and 0.13 ml 11.6 N HCl. Viscosities for crude culture broths and semi-purified xanthan gum were determined. As in Example 1, partial purification of xanthan was by precipitation of polysaccharide by addition of two volumes of isopropyl alcohol and collection of the precipitation on a GFA filter followed by drying and weighing. The results are tabulated below:

**Table 3**

| Host | Plasmid | Yield (g xanthan/l) | Viscosity (cps at 1 rpm)^{a} | |
|---|---|---|---|---|
| | | | Fermentation Broth | 0.5% (w/v) Semi-purified Xanthan |
| X59 | pRK311 | 14 | 620 | 340 |
| | c8 | 17 | 1000 | 300 |
| | c8C | 15 | 1300 | 480 |
| | c8D | 14 | 420 | 110 |
| | c1021 | -- | -- | -- |
| | c1020 | 12 | 140 | 270 |
| | c1042 | 17 | 500 | 170 |
| | c1041 | 17 | 710 | 320 |
| | | | | |
| X50 | pRK311 | 16 | 1700 | 420 |
| | c8 | 17 | 2100 | 460 |
| | c8C | 17 | 3200 | 800 |
| | c8D | 14 | 930 | 290 |
| | c1021 | 13 | 880 | 320 |
| | c1020 | 15 | 420 | 300 |
| | c1042 | 18 | 960 | 270 |

| | | | | |
|---|---|---|---|---|
| ^{a} Brookfield LV viscometer with spindle number 18 or 31. Fermentation broths were diluted 1:1 with 0.1 M NaCl prior to measuring viscosities. | | | | |

Subclone c8C accumulates as much xanthan in the culture broth as the parent clone c8; however, the product has an unexpected higher viscosity per weight of semi-pure material. Thus, the cloning and reintroduction of cloned DNA into X. campestris affects both quantity and quality of xanthan, and improved viscosity (or other properties) can be obtained routinely by selecting subclones having the desired property.

### Example 3

### Direct Utilization of Lactose in Clarified Cheese Whey for Xanthan Gum Synthesis

In this example we describe the construction or a plasmid vector that is useful for integrating foreign DNA into the chromosome of X. campestris. Using this vector we inserted the lac genes from pGC9114 (RP1::Tn951) into a rifampicin-resistant derivative of X. campestris B1459. The genetic stability of lactose utilization and conversion of lactose or lactose in clarified whey to xanthan gum was determined. In addition, a preliminary characterization of the quality of the xanthan gum made by this strain from clarified cheese whey is described.

### Materials and Methods

### Bacterial Strains, Plasmids and Growth Conditions

Some materials are described in Example 1. X. campestris B1459S-4L-II (our strain X55) was obtained from the Northern Regional Research Center in Peoria, Illinois. E. coli strain MC1009 (Δlac ipozy-X74, galK, galU, Δara-leu-7697, strA, recA) was obtained from J. Hoch and strain JC3272 (his, trp, lys, Δlac ipozy-X74, strA) containing plasmid pGC9114 (RP1::Tn951) from G. Somkuti. Plasmid pRK290 was obtained from D. Helinski (Ditta et al., Proc. Natl. Acad. Sci. USA (1980) 77:7347-7351). Xanthomonas strains were cultured at 30°C in four related liquid or solid (with agar) media: YT (10 g/l Difco yeast extract, 16 g/l Difco tryptone, 5 g/l NaCl); YTS (5 g/l Difco yeast extract, 5 g/l Difco tryptone, 3.5 g/l K₂HPO₄, 2.6 g/l KH₂PO₄, 0.26 g/l MgSO₄·7H₂O, 6 mg/l H₃BO₃, 6 mg/l ZnO, 2.6 mg/l FeCl₃·6H₂O, 20 mg/l CaCO₃); YPS (with an equal weight of peptone substituted for tryptone in YTS); PS (10 g/l peptone substituted for yeast extract and tryptone in YTS); S (2 to 4 g/l [NH₄]₂SO₄ substituted for yeast extract and tryptone in YTS). The volume of culture was always one-tenth to one-fifth the flask capacity. E. coil strains were grown in LB broth or YT. Antibiotics and carbohydrate were added as needed. Whey was "sweet whey" from Sigma. It was 65% lactose by dry weight, 13% protein, 8% ash and 2% lactic acid. A 30% (w/v) solution was autoclaved at 121°C for 20 min and centrifuged to clarify. The pH before autoclaving and after clarification was about 6. The phenol-H₂SO₄ assay was used to measure final lactose concentration.

### DNA Preparation and Analysis

See Maniatis et al., Molecular Cloning: A Laboratory Manual (1982) Cold Spring Harbor Laboratory, Cold Spring Harbor, New York, for standard cloning techniques. DNA was prepared by the boiling method or the Birnboim and Doly procedure (Nucleic Acids Res. (1979) 1:1513-1523) and when necessary purified by equilibrium sedimentation in density gradients of CsCl containing ethidium bromide. Restriction enzymes and DNA ligase were used according to the instructions of the manufacturer. Transformation of E. coli cells with plasmids or ligation mixtures was standard and conjugal transfer of plasmids into X. campestris follows the tri-parental mating scheme (Ditta et al., Proc. Natl. Acad. Sci. USA (1980) 77:7347-7351).

### Xanthan Gum Isolation and Analysis

In order to measure amounts of xanthan gum, culture samples (without prior removal of cells) were added to two volumes of isopropyl alcohol. The precipitated material was collected by filtration onto What-man 934-AH filters, then dried at 80°C in a vacuum oven and weighed. For viscosity measurements the dried precipitate was ground in a mortar and sieved through a 250 micron mesh before resuspending in 0.1% (w/v) NaCl. Viscosity measurements over a range of shear rates at room temperature were made with a Brookfield LVT viscometer. Protein concentrations were determined with the BioRad Protein Assay and standards of bovine serum albumin (Sigma).

### Results

### Construction of Lactose-positive X. campestris

Plasmid pGC9114 is a derivative of plasmid RP1 and carries Tn951, a transposon that confers lactose-utilization. We verified that a subfragment of pGC9114 of about 10.5 kbp and flanked by BamHI restriction sites carried the lac genes. We subcloned that fragment to pUC13 and transformed Lac⁻ E. coli MC1009 to Lac⁺ (blue colonies on nutrient plates containing XgaI and IPTG). The same 10.5 kbp fragment was subcloned into a plasmid "integration" vector (pSY1181) that could be conjugally transferred from E. coli to X. campestris but could not replicate in the latter. We call this an "integration" vector since the only way that the lac genes can be stably maintained in the recipient is if they recombine with and become integrated into the bacterial chromosome. The four steps in the construction of plasmids pSY1181 and pSY1232 are diagrammed in Figure 5 and explained below.

To promote integration into the chromosome we included a fragment of chromosomal DNA in pSY1232. Isolated fragments of the X. campestris wild-type chromosome that complemented or restored xanthan gum synthesis to mutants unable to make the polysaccharide are described in Example 1. Colonies of both the wild-type and mutants carrying the "xanthan" genes cloned on cosmid vectors were mucoid, while the mutants alone were non-mucoid. One such clone was c1, a recombinant between the cosmid vector pRK311 and an approximately 22 kbp chromosomal fragment. A derivative of c1, which carried transposon Tn5 (kanamycin-resistance) in a site within c1 but which did not inactivate complementation by c1 for the corresponding mutant m1, was our starting material. As shown by Step 1 of Figure 5, this plasmid was restricted with BamHI enzyme and recircularized to create a single BamHI cloning site flanked by the c1 complementing DNA and the kanamycin-resistance gene of Tn5. The c1-Kan region is bounded by HindIII sites.

The second step was to convert the matable broad host range plasmid pRK290 to narrow host range by substituting the origin of replication from pUC13 for the oriV of pRK290. The third step was to fuse c1-Kan with pRKpUC via the HindIII sites to create the "integration" vector pSY1181. The last step was to insert the lac genes at the BamHI site of pSY1181 to generate pSY1232. E. coli MC1009 transformed with pSY1232 are resistant to ampicillin and kanamycin and give blue colonies on plates containing Xgal and IPTG.

In order to allow X. campestris to utilize lactose we transferred pSY1232 into strain X59 using a triparental conjugation scheme with pRK2013 as the helper plasmid (Ditta et al., op. cit.). Exconjugants were initially selected on kanamycin since X. campestris is naturally resistant to ampicillin, the other resistance gene carried on pSY1232. All the Kan^{R} exconjugants were then shown to grow on minimal plates with lactose, unlike X59. The Kan^{R} Lac⁺ exconjugants were indistinguishable, and one, named X59-1232, was chosen as representative for further work. Similar results were obtained by mobilizing the Lac⁺ plasmid pGC9114 into X59. In liquid cultures we found that the plasmid-bearing X59-pGC9114 grew more slowly than either X59 or X59-1232, which grew at similar rates. We tentatively attributed this slower growth to the "cost" of maintaining the multi-copy plasmid.

We immediately noticed that in the absence of tetracycline selection the plasmid pGC9114 was lost from a culture of X59, whereas in the absence of kanamycin selection for strain X59-1232 the cryptic marker was retained. More importantly the ability to utilize lactose behaved in the same way. This was consistent with there being at least part of the pSY1232 DNA stably integrated in the bacterial chromosome. By DNA hybridization analysis, we confirmed that the narrow host range plasmid had integrated into the chromosome (data not shown). Furthermore, the restriction fragment sizes were consistent with insertion into the c1 chromosomal region. Similarly, the vector pSY1181 also integrates in this region, so that the strain becomes resistant to kanamycin.

### Stability of Integrated Lactose Genes

Since the overall objective was to generate a stable strain for converting lactose to xanthan gum, we measured stability for this trait after serially sub-culturing X59-pGC9114 and X59-1232 for many generations without tetracycline for selection of plasmid pGC9114 or kanamycin in the case of X59-1232. In either case, the X59 host is resistant to rifampicin. This allows a counterselection for rifampicin-sensitive accidental contamination during repeated serial transfer. Each strain was grown both in glucose and lactose, and the ratio of the amount of xanthan produced from lactose to glucose was calculated. The results are given in Table 4. The ability to convert lactose to xanthan gum by the plasmid bearing strain, X59pGC9114, decreased to half its original level at the third passage. In contrast, X59-1232 carrying the lac genes integrated into the chromosome showed stable conversion of lactose to xanthan gum through the end of the parallel experiment, a total of 42 generations.

**Table 4**

| Genetic Stability of Utilization of Lactose for Xanthan Gum Synthesis | | | | | | | |
|---|---|---|---|---|---|---|---|
| Passage Number^{a} | Generation Number^{b} | Xanthan Gum (weight percent) | | | | | |
| | | X59pGC9114 | | | X59-1232 | | |
| | | Lac | Glc | Lac/Glc | Lac | Glc | Lac/Glc |
| 0 | 7 | 1.6 | 2.0 | 0.8 | 1.6 | 1.9 | 0.8 |
| 1 | 14 | 1.7 | 2.0 | 0.9 | 1.7 | 2.0 | 0.9 |
| 3 | 28 | 0.6 | 1.5 | 0.4 | 1.5 | 1.7 | 0.9 |
| 4 | 35 | 0.1 | 1.8 | 0.1 | 1.5 | 2.0 | 0.8 |
| 5 | 42 | 0.2 | 1.7 | 0.1 | 1.7 | 2.2 | 0.8 |

| | | | | | | | |
|---|---|---|---|---|---|---|---|
| ^{a} Initial inocula were grown in YT plus rifampicin (50 µg/ml) with tetracycline (7.5 µg/ml) for X59pGC9114 or kanamycin (50 µg/ml) for X59-1232. Each passage was in YT plus rifampicin with an inoculum of 10⁷ cells/ml and was ended at about 10⁹ cells/ml (O. D. 600 = 1). After each passage, shake flasks YPS medium with either lactose or glucose at 2% (w/v) were inoculated with 10⁷ cell/ml. After 48 hrs the amount of xanthan gum in each flask was measured by precipitation with 2 volumes of isopropyl alcohol and then dried and weighed. | | | | | | | |
| ^{b} Includes about 7 generations per passage and about 7 generations during carbohydrate conversion assay. | | | | | | | |

### Utilization of Carbohydrate Substrate for Xanthan Gum Synthesis

Parallel shake flask cultures of strains X59 (Lac⁻) and X59-1232 (Lac⁺) were tested for utilization of carbohydrate for the synthesis of xanthan gum. Exopolysaccharide accumulation was measured with glucose, lactose and clarified cheese whey at equivalent weight percents of glucose or lactose. The results are given in Table 5. The Lac⁻ parental strain X59 did not convert appreciable lactose or lactose in clarified whey to xanthan gum, compared to the stable Lac⁺ strain X59-1232. Since the residual amounts of substrates from the carbohydrate, yeast extract, tryptone and whey were not determined, we could not calculate the absolute conversion efficiencies. However, the amounts of xanthan gum shown in Table 7 are similar to those of our most productive strains of X. campestris, which can convert over 70% of substrate to xanthan during controlled fermentations.

**Table 5**

| Utilization of Carbohydrate Substrate for Xanthan Gum Synthesis | | | |
|---|---|---|---|
| Strain | Carbohydrate Substrate | Xanthan gum (weight percent)^{a} | |
| | | 12 Hrs | 24 Hrs |
| X59 (Lac⁻) | glucose | 1.2 | 2.1 |
| | lactose | 0.2 | 0.2 |
| | whey lactose | 0.0 | 0.4 |
| | | | |
| X59-1232 (Lac⁺) | glucose | 1.1 | 2.0 |
| | lactose | 1.6 | 2.0 |
| | whey lactose | 1.7 | 1.8 |

| | | | |
|---|---|---|---|
| ^{a} Inocula were grown in YT medium with rifampicin (50 µg/ml), centrifuged, washed with LB broth and resuspended at 2x10⁹ cells/ml in YTS medium plus carbohydrate substrate at 2% (w/v). Samples were withdrawn and xanthan was precipitated with 2 volumes of isopropyl alcohol, and then dried and weighed. | | | |

### Quality of Xanthan Gum Produced from Glucose, Lactose and Clarified Cheese Whey

The following cultures were grown in shake flasks containing 200 ml of PS medium supplemented with the indicated carbohydrate at 2% (w/v): strain X59, glucose; X59-1232, lactose; X59-1232, clarified cheese whey (lactose). After 48 hrs growth the culture contents were precipitated with 2 volumes of isopropyl alcohol, dried and ground to uniform particle size (about 100-200 microns). Samples of each were resuspended in 0.1% (w/v) NaCl at specific weight percentages, with the weights determined to the exclusion of water, protein and ash. Viscosities were measured over a range of shear rates and the results are given in Figure 6. The solution viscosities for the xanthan-containing material made by X59 from glucose or X59-1232 from lactose were not distinguishable. However, the material made in the presence of clarified cheese whey appeared to be less viscous, requiring almost twice as much by weight to give equal viscosity. A subsequent mixing experiment indicated that an unknown clarified whey component lowers the viscosity of xanthan gum. We prepared a separate culture of X59-1232 grown on PS medium plus lactose. The xanthan-containing material was precipitated either in the presence or absence of added clarified whey. Enough clarified whey was added to make the final lactose concentration 2% (w/v). The resulting viscosities from this mixing experiment are superimposed on Figure 6. Most of the apparent qualitative difference is accounted for by the whey effect on viscosity.

## Claims

1. A Xanthomonas campestris strain in biologically pure culture, which strain:
(i) produces xanthan gum in submerged culture, and
(ii) exhibits stable, enhanced lactose utilization activity, which enhanced activity is conferred by exogenous genetic information containing lactose utilization genes integrated into the bacterial chromosome, said exogenous genetic material comprising exogenous DNA which controls the synthesis of xanthan, and has a restriction map of Figure 1 or a segment thereof, which exogenous DNA is contained in E. coli strains having deposit numbers ATCC67386, ATTC67387, ATTC67388.

2. The strain of claim 1, wherein the exogenous DNA which controls the synthesis of xanthan is capable of complementing a xanthan gum synthesis negative mutation, and has a restriction map of segment c1H5, c1, c9H7, c82, c9 or c9e of Figure 1,
wherein the xanthan gum production of the strain is higher relative to the xanthan gum production obtained under the same conditions using a Xanthomonas campestris strain not containing this exogenous DNA.

3. The strain of claim 1 which is capable of producing at least one gram of xanthan per litre of culture medium per hour.

4. The strain of any one of claims 1 to 3 wherein said strain is not mucoid in a culture medium lacking glucose and mucoid in a culture medium containing glucose.

5. A process for obtaining the strain of claim 1 which process comprises use of a plasmid which includes:
(i) the lactose utilization genes and,
(ii) a DNA sequence which is homologous to the bacterial chromosome, wherein the plasmid contains the DNA sequence designated c1 of Figure 1.

6. A process as claimed in claim 5 wherein the plasmid is plasmid pSY1232 shown in Fig 5.

7. A method for xanthan gum production comprising culturing a Xanthomonas campestris strain in a culture medium, which strain:
(i) has a xanthan-increasing modification which is exogenous genetic information from Xanthomonas campestris controlling the synthesis of xanthan integrated into the bacterial chromosome, and
(ii) has increased lactase activity relative to a parent of said strain,
wherein said exogenous genetic information comprises lactose utilization genes responsible for said increased lactase activity and comprises a sequence having a restriction map of Figure 1, 3 or a segment thereof, which sequence is contained in E. coli strains having deposit numbers ATCC67386, ATTC67387, ATTC67388.

8. The method of claim 7, wherein the exogenous DNA which controls the synthesis of xanthan:
(i) is capable of complementing a xanthan gum synthesis negative mutation, and
(ii) has a restriction map of segment c1H5, c1, c9H7, c82, c9 or c9e of Figure 1
and wherein the xanthan gum production by the strain is higher relative to the xanthan gum production obtained under the same conditions using a Xanthomonas campestris strain not containing this exogenous DNA.

9. The method of claim 7 wherein the xanthan is isolated from the culture medium.

10. The method of claim 7 wherein said strain is capable of producing at least 1 gram of xanthan per litre of culture medium per hour.

11. The method of claim 7 where the strain is cultured in a culture medium containing lactose.

12. Plasmid pSY1232 shown in Fig 5.

## Patentansprüche

1. Xanthomonas campestris-Stamm in biologisch reiner Kultur, wobei der Stamm:
(i) in Submerskultur Xanthanlösung produziert und
(ii) stabile, gesteigerte Lactose-Verwertungsaktivität aufweist, wobei die gesteigerte Aktivität durch in das Bakterienchromosom integrierte, exogene, genetische Information verliehen wird, welche Lactose-Verwertungsgene enthält, wobei das exogene genetische Material die Synthese von Xanthan regelnde, exogene DNA umfasst, und eine Restriktionskarte wie in Fig. 1 oder ein Segment davon aufweist, wobei die exogene DNA in E. coli-Stämmen mit den Hinterlegungsnummern ATCC67386, ATCC67387 und ATCC67388 enthalten ist.

2. Stamm nach Anspruch 1, worin die exogene DNA, welche die Synthese von Xanthan regelt, zur Komplementierung einer negativen Xanthanlösungs-Synthese-Mutation fähig ist und eine Restriktionskarte von Segment c1H5, c1, c9H7, c82, c9 oder c9e aus Fig. 1 aufweist, worin die Xanthanlösungs-Produktion des Stammes höher ist als die Xanthanlösungs-Produktion, die unter denselben Bedingungen unter Verwendung eines Xanthomas campestris-Stamms erzielt wird, der diese exogene DNA nicht enthält.

3. Stamm nach Anspruch 1, der zur Produktion von zumindest einem Gramm Xanthan pro Liter Nährmedium pro Stunde fähig ist.

4. Stamm nach einem der Ansprüche 1 bis 3, worin der Stamm in einem Glucosefreien Nährmedium nicht mukoid und in einem Glucose-hältigen Nährmedium mukoid ist.

5. Verfahren zum Erhalt des Stamms nach Anspruch 1, wobei das Verfahren die Verwendung eines Plasmids umfasst, welches
(i) die Lactose-Verwertungsgene und
(ii) eine DNA-Sequenz umfasst, die homolog zum Bakterienchromosom ist, worin das Plasmid die mit c1 bezeichnete DNA-Sequenz aus Fig. 1 enthält.

6. Verfahren nach Anspruch 5, worin das Plasmid das in Fig. 5 dargestellte Plasmid pSy1232 ist.

7. Verfahren zur Herstellung von Xanthanlösung, das die Kultivierung eines Xanthomas campestris-Stamms in einem Nährmedium umfasst, wobei der Stamm:
(i) eine Xanthan-steigernde Modifikation aufweist, die eine in das Bakterienchromosom integrierte, exogene, genetische Information von Xanthomas campestris ist, welche die Synthese von Xanthan regelt, und
(ii) relativ zu Vorfahren des Stamms gesteigerte Lactase-Aktivität aufweist,
worin die exogene genetische Information Lactose-Verwertungsgene umfasst, die für die gesteigerte Lactase-Aktivität verantwortlich sind, und eine Sequenz mit einer Restriktionskarte von Fig. 1, 3 oder ein Segment davon umfasst, wobei die Sequenz in E. coli-Stämmen mit den Hinterlegungsnummern ATCC67386, ATCC67387 und ATCC 67388 enthalten ist.

8. Verfahren nach Anspruch 7, worin die exogene DNA, welche die Synthese von Xanthan regelt,
(i) zur Komplementierung einer negativen Xanthanlösungs-Synthese-Mutation fähig ist und
(ii) eine Restriktionskarte von Segment c1 H5, c1, c9H7, c82, c9 oder c9e aus Fig. 1 aufweist,
und worin die Xanthanlösungs-Produktion des Stammes höher ist als die Xanthanlösungs-Produktion, die unter denselben Bedingungen unter Verwendung eines Xanthomas campestris-Stamms erzielt wird, der diese exogene DNA nicht enthält.

9. Verfahren nach Anspruch 7, worin das Xanthan aus dem Nährmedium isoliert wird.

10. Verfahren nach Anspruch 7, worin der Stamm zur Produktion von zumindest einem Gramm Xanthan pro Liter Nährmedium pro Stunde fähig ist.

11. Verfahren nach Anspruch 7, worin der Stamm in einem Lactose-hältigen Nährmedium kultiviert wird.

12. Das in Fig. 5 dargestellte Plasmid pSY1232.

## Revendications

1. Souche de Xanthomonas campestris en culture biologiquement pure, laquelle souche:
(i) produit de la gomme xanthane en culture submergée, et
(ii) présente une activité d'utilisation du lactose améliorée et stable, laquelle activité améliorée est conferée par des gènes d'utilisation du lactose contenant une information génétique exogène intégrés dans le chromosome bactérien, ledit matériel génétique exogène comprenant de l'ADN exogène qui contrôle la synthèse du xanthane et a une carte de restriction de la figure 1 ou un segment de celle-ci, lequel ADN exogène est contenu dans des souches de E. coli ayant les numéros de dépôt ATCC67386, ATTC67387, ATTC67388.

2. Souche de la revendication 1 où l'ADN exogène qui contrôle la synthèse du xanthane est capable de compléter une mutation négative de synthèse de gomme xanthane et a une carte de restriction du segment c1H5, c1, c9H7, c82, c9 ou c9e de la figure 1, où la production de gomme xanthane de la souche est plus élevée relativement à la production de gomme xanthane obtenue dans les mêmes conditions en utilisant une souche de Xanthomonas campestris ne contenant pas cet ADN exogène.

3. Souche de la revendication 1 qui est capable de produire au moins un gramme de xanthane par litre de milieu de culture par heure.

4. Souche de l'une quelconque des revendications 1 à 3 où ladite souche n'est pas mucoïde dans un milieu de culture manquant de glucose et mucoïde dans un milieu de culture contenant du glucose.

5. Procédé pour obtenir la souche de la revendication 1, lequel procédé comprend l'utilisation d'un plasmide qui contient:
(i) les gènes d'utilisation du lactose et,
(ii) une séquence d'ADN qui est homologue avec le chromosome bactérien, où le plasmide contient la séquence d'ADN désignée par c1 sur la figure 1.

6. Procédé selon la revendication 5 où le plasmide est le plasmide pSY1232 montré à la figure 5.

7. Méthode pour la production de gomme xanthane comprenant la culture d'une souche de Xanthomonas campestris dans un milieu de culture, laquelle souche:
(i) a une modification augmentant le xanthane qui est une information génétique exogène de Xanthomonas campestris contrôlant la synthèse du xanthane intégré dans le chromosome bactérien, et
(ii) a une activité accrue de lactase relativement à un parent de ladite souche,
où ladite information génétique exogène comprend des gènes d'utilisation du lactose responsables de ladite activité accrue de lactase et comprend une séquence ayant une carte de restriction de la figure 1, 3 ou son segment, laquelle séquence est contenue dans les souches de E. coli ayant les numéros de dépôt ATCC67386, ATTC67387, ATTC67388.

8. Méthode de la revendication 7 où l'ADN exogène qui contrôle la synthèse du xanthane:
(i) est capable de compléter une mutation négative de la synthèse de la gomme xanthane, et
(ii) a une carte de restriction du segment c1H5, cl, c9H7, c82, c9 ou c9e de la Figure 1
et où la production de gomme xanthane par la souche est plus élevée relativement à la production de gomme xanthane obtenue dans les mêmes conditions en utilisant une souche de Xanthomonas campestris ne contenant pas cet ADN exogène.

9. Méthode de la revendication 7 où le xanthane est isolé du milieu de culture.

10. Méthode de la revendication 7 où ladite souche est capable de produire au moins 1 gramme de xanthane par litre du milieu de culture par heure.

11. Méthode de la revendication 7 où la souche est cultivée dans un milieu de culture contenant du lactose.

12. Plasmide pSY1232 montré à la figure 5.
